# FASCICULE DE BREVET EUROPEEN

(11) **EP 3 549 905 B1**
(45) Date de publication et mention de la délivrance du brevet: **03.11.2021**
(21) Numéro de dépôt: 19158130.5
(22) Date de dépôt: 28.03.2011
(51) Int. Cl.: B81B 1/00

(54) **DISPOSITIF DE FORMATION DE GOUTTES DANS UN CIRCUIT MICROFLUIDIQUE**
VORRICHTUNG ZUM BILDEN VON TROPFEN IN EINER MIKROFLUIDISCHEN SCHALTUNG
DEVICE FOR FORMING DROPS IN A MICROFLUIDIC CIRCUIT

(30) Priorité: 30.03.2010 FR 1001298
(43) Date de publication de la demande: 09.10.2019
(62) Demande divisionnaire de: 11719305.2
(73) Titulaire: Centre National de la Recherche Scientifique, 75794 Paris Cedex 16 (FR); Ecole Polytechnique, 91128 Palaiseau Cedex (FR)
(72) Inventeur: BAROUD, Charles, 75013 PARIS (FR); DANGLA, Rémi, 75017 PARIS (FR)
(74) Mandataire: Nony

(56) Documents cités:
- WO-A2-2007/133710
- WO-A2-2009/048532
- WO-A2-2010/033200
- US-A1- 2006 051 329
- US-A1- 2009 098 168
- YUNG-CHIEH TAN E A: "Controlled Microfluidic Encapsulation of Cells, Proteins, and Microbeads in Lipid Vesicles", JOURNAL OF THE AMERICAN CHEMICAL SOCIETY, AMERICAN CHEMICAL SOCIETY, US, vol. 128, no. 17, 5 avril 2006 (2006-04-05), pages 5656-5658, XP002530562, ISSN: 0002-7863, DOI: DOI:10.1021/JA056641H [extrait le 2006-04-05]
- CORDERO M-L ET AL: "Holographic control of droplet microfluidics", PROCEEDINGS OF THE SPIE, SPIE, BELLINGHAM, VA, US, vol. 7038, 10 août 2008 (2008-08-10), pages 70381J-1, XP007910334, ISSN: 0277-786X, DOI: DOI:10.1117/12.794855
- MICHELE ZAGNONI; E.A.: "Electrically initiated upstream coalescence cascade of droplets in a microfluidic flow", PHYSICAL REVIEW E, vol. 80, no. 4, octobre 2009 (2009-10), pages 046303-1-046303-9, XP002611761,
- CORDERO MARIA LUISA ET AL: "Thermocapillary manipulation of droplets using holographic beam shaping: Microfluidic pin ball", APPLIED PHYSICS LETTERS, AIP, AMERICAN INSTITUTE OF PHYSICS, MELVILLE, NY, US, vol. 93, no. 3, 24 juillet 2008 (2008-07-24), pages 34107-34107, XP012113187, ISSN: 0003-6951, DOI: DOI:10.1063/1.2952374

## Description

La présente invention concerne un dispositif de formation de gouttes dans un circuit microfluidique, en particulier de microgouttes et de nanogouttes, dont la taille varie de quelques centaines de nanomètres à quelques centaines de microns.

De telles gouttes sont utilisées dans plusieurs domaines techniques. Pour chaque domaine, les méthodes de formation des gouttes sont différentes.

Un premier domaine technique concerne les applications de laboratoire sur puce ou autres biotechnologies. Dans ce domaine, une première approche consiste à utiliser un dispositif comportant au moins un microcanal d'écoulement d'un premier fluide, également appelé fluide porteur, dans lequel débouche perpendiculairement au moins un second microcanal d'écoulement d'un second fluide, non miscible avec le premier fluide. Le premier fluide (généralement de l'huile) cisaille le second fluide (généralement de l'eau pour les applications biologiques) de manière à former des gouttes de second fluide qui sont ensuite transportées par le premier fluide. Les débits des deux fluides et la géométrie des microcanaux sont ajustés de manière à obtenir une taille et une fréquence désirées de formation de gouttes, qui dépendent aussi des viscosités des deux fluides.

Les dispositifs de ce type comportent nécessairement des moyens de forçage tels qu'une pompe, permettant de faire circuler les deux fluides. La taille des gouttes étant fonction du débit de chaque fluide, il est nécessaire d'ajuster précisément les débits des fluides, ce qui peut rendre délicate la mise en œuvre de ces dispositifs.

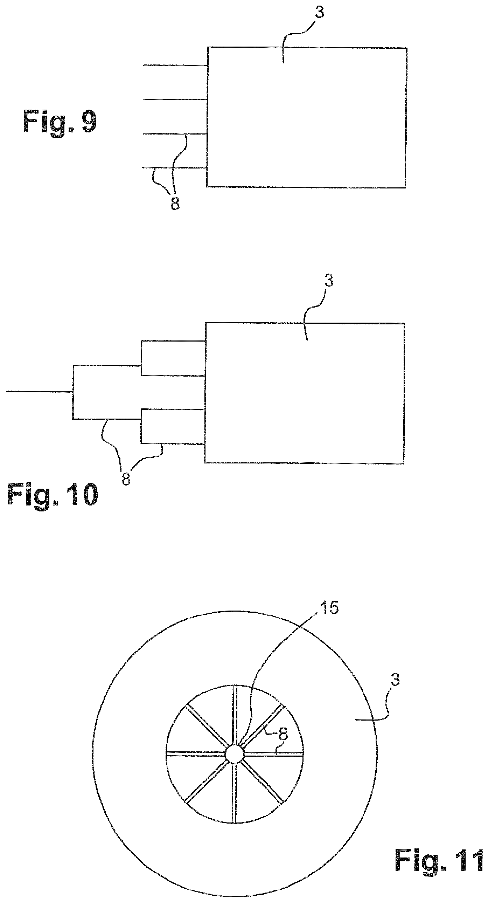

Par exemple, le document US 2006/0051329 décrit un dispositif d'encapsulation de gouttes contenant des cellules, comportant un premier canal d'arrivée d'un débit du fluide contenant les cellules, présentant un évasement en partie aval (voir figure 9), et un second canal de circulation d'huile croisant perpendiculairement le premier canal, le débit d'huile cisaillant le débit de fluide contenant les cellules afin de former des gouttes. Un dispositif similaire est présenté dans le document « Controlled Microfluidic Encapsulation of Cells, Proteins, and Microbeads in Lipid Vesicles », YUNG-CHIEH TAN et al., Journal of the American Chemical Society, vol. 128, no. 17, 5 avril 2006, pages 5656-5658.

Le document WO 2009/048532 présente également un dispositif de formation de gouttes comportant un premier canal d'arrivée de gaz, dans lequel débouchent deux canaux latéraux opposés d'arrivée d'eau, de manière à former des bulles de gaz entourées d'eau, et deux autres canaux latéraux d'arrivée d'huile permettant d'encapsuler l'ensemble. Les bulles sont formées par cisaillement d'un premier débit de fluide (gaz), à l'aide d'un autre débit de fluide (eau).

D'autres dispositifs de formation de gouttes par cisaillement d'un débit de fluide sont connus des documents WO 2010/033200, WO 2007/133710, « Holographie control of droplet microfluidics », CORDERO M-L et al., Proceedings of the SPIE, vol. 7038, 10 août 2008, pages 70381 J-1, « Electrically initiated upstream coalescence cascade of droplets in a microfluidic flow », MICHELE ZAGNONI et al., PHYSICAL REVIEW E., vol. 80, no. 4, octobre 2009, pages 046303-1 -046303-9, et « Thermocapillary manipulation of droplets using holographie beam shaping : Microfluidic pin bail », CORDERO M-L et al., APPLIED PHYSICS LETTERS, vol. 93, no. 3, 24 juillet 2008, page 34107.

Le document US 2009/0098168 divulgue un dispositif de formation de gouttes comportant un canal d'arrivée d'un débit de fluide débouchant dans une buse d'expansion au travers d'un orifice. La buse comporte deux parois divergentes et contient un fluide distinct de celui circulant dans le canal en amont de l'orifice. Les gouttes sont formées du fait du pincement hydrodynamique à l'orifice, alors que les parois divergentes assurent l'uniformité de l'émulsion. Le système force un courant central d'une phase dispersée et deux courants tubulaires latéraux à travers l'orifice dans une seconde chambre, la convergence de l'écoulement environnant de liquide fractionnant le filet à l'orifice.

Une deuxième approche est celle de la microfluidique dite "digitale", dans laquelle les gouttes sont typiquement formées par électromouillage, en appliquant des tensions électriques différentes à différentes parties des gouttes.

La taille des gouttes formées à l'aide de cette technique est bien supérieure à celle des nanogouttes ou des microgouttes. Cette technique pose en outre le problème de la contamination entre gouttes et de l'évaporation des gouttes.

Enfin, il existe plusieurs approches pour produire des gouttes à la demande en éjectant rapidement du liquide à travers une aiguille ou un trou, à l'aide de dispositifs s'apparentant souvent aux systèmes d'imprimantes à jet d'encre, qui produisent des gouttes qui impactent une surface avec beaucoup d'énergie et qui génèrent des éclaboussures. Ces dispositifs nécessitent de plus des moyens techniques coûteux, comme une source de haute tension ou des moteurs de précision.

Un second domaine technique concerne la science des matériaux, dans lequel plusieurs approches ont été développées afin de produire des mousses ou des émulsions, et donc des populations de bulles ou de gouttes. Les applications sont diverses et concernent notamment l'industrie alimentaire et l'industrie cosmétique.

D'autres approches consistent à encapsuler des gouttes dans d'autres gouttes. Par exemple, une goutte d'eau peut être encapsulée dans une goutte d'huile, qui est elle-même contenue dans de l'eau. Toutes ces approches nécessitent l'utilisation de moyens de forçage coûteux et difficiles à mettre en œuvre.

En outre, d'une manière générale, on vise à augmenter les débits de gouttes produites, tout en garantissant l'obtention de gouttes ou de bulles monodispersées, c'est-à-dire présentant une taille constante et contrôlée.

L'invention a notamment pour but d'apporter une solution simple, efficace et économique à ces problèmes.

A cet effet, elle propose un dispositif de formation de gouttes dans un circuit microfluidique selon la revendication 1.

Dans ce dispositif, le second fluide est soumis, au niveau du débouché du microcanal dans la chambre, à deux forces antagonistes, dues à la tension de surface. Une première force est un gradient d'énergie de surface qui est dû au changement de la surface de la goutte quand elle se forme et qui tend à extraire le second fluide hors du microcanal, de manière à former un "doigt" de second fluide faisant saillie dans la chambre et rattaché au second fluide contenu dans le microcanal, puis à former une goutte en séparant le doigt du second fluide contenu dans le microcanal.

Une seconde force, agissant en sens contraire de la première et correspondant à la force capillaire, tend à maintenir le doigt de second fluide attaché au second fluide contenu dans le microcanal.

Le doigt précité se détache du second fluide contenu dans le microcanal lorsque la première force devient supérieure à la seconde force. Or, la première force est notamment fonction, pour une géométrie donnée du microcanal et de la chambre, du volume du doigt de second fluide. Ainsi, en fonctionnement, le volume du doigt va augmenter progressivement, jusqu'à ce que la première force devienne supérieure à la seconde force et que le doigt se détache pour former une goutte.

La goutte est ensuite transportée par l'augmentation de section de la chambre, de l'amont vers l'aval.

On remarque qu'il n'est pas nécessaire que les premier et second fluide soient en circulation, l'important étant uniquement que le second fluide soit amené jusqu'au débouché du microcanal dans la chambre. Il n'est donc pas nécessaire de prévoir des moyens de forçage des différents fluides. Le transport des gouttes du second fluide dans la chambre résulte de l'augmentation de la section de passage. En effet, une goutte située dans une zone de faible section, dans laquelle elle a une forme écrasée, sera naturellement attirée par une zone de plus grande section, dans laquelle elle peut prendre une forme plus sphérique.

De plus, la taille des gouttes est sensiblement indépendante du débit du second fluide. Elle est essentiellement fonction de la section d'amenée du second fluide à l'entrée de la chambre et de la divergence desdites parois opposées de la chambre, c'est-à-dire fonction de paramètres géométriques figés et invariables dans le temps, la taille des gouttes étant ainsi calibrée avec précision.

La taille des gouttes ne dépend pas non plus de la tension de surface, car la même tension de surface agit à la fois pour détacher les gouttes et pour les retenir. De cette façon, la taille des gouttes est indépendante de la nature exacte des fluides ou de leur éventuelle contamination, et ne dépend que très faiblement de la viscosité des fluides.

Enfin, la taille des gouttes n'est pas non plus influencée par la géométrie des parois situées à distance du débouché des microcanaux, de sorte que différentes formes de chambre peuvent être utilisées.

La chambre utilisée a par exemple une section sensiblement rectangulaire dont la hauteur est comprise entre les deux parois opposées divergentes et dont la longueur est grande par rapport à la hauteur.

La longueur est ainsi par exemple supérieure à 10 fois la hauteur.

Bien entendu, la chambre peut présenter d'autres formes. En particulier, les parois de la chambre peuvent diverger dans plus d'une direction. A titre d'exemple, la chambre peut présenter une forme sphérique ou ovoïde.

De manière préférée, la hauteur de la chambre au débouché des microcanaux est inférieure au diamètre des gouttes à former.

En variante, l'une des parois de la chambre comporte une marche, une partie concave ou une partie convexe au débouché des microcanaux.

Ces variations de la géométrie à l'entrée de la chambre permettent de contrôler la taille ou la vitesse de déplacement des gouttes. C'est ainsi que la présence d'une marche permet de former des gouttes plus petites, qu'une partie concave permet de diminuer la vitesse de déplacement des gouttes après leur formation et qu'une partie convexe permet de mieux calibrer la taille des gouttes.

Dans un premier mode de réalisation, le débit du premier fluide dans la chambre est sensiblement nul.

Dans une variante de réalisation, le débit du premier fluide dans la chambre est réglé à une valeur déterminée.

La divergence des deux parois opposées de la chambre correspond par exemple à une pente comprise entre 1 et 4 % environ d'une paroi par rapport à l'autre.

Bien entendu, ces valeurs ne sont données qu'à titre d'exemple, et la pente peut avoir une valeur infinitésimale ou une valeur de 100 %, correspondant à une paroi verticale par rapport à une paroi horizontale.

Selon une autre caractéristique de l'invention, le dispositif comporte des moyens pour modifier localement la tension de surface des seconds fluides.

Ceci permet notamment d'ajuster la taille des gouttes produites par rapport à la taille qu'elles auraient sans modification de la tension de surface.

Dans une réalisation de l'invention, les moyens de modification de la tension de surface des seconds fluides comportent des moyens de chauffage des seconds fluides, par exemple par un faisceau laser appliqué localement ou par des électrodes intégrées au circuit microfluidique ou en utilisant un autre moyen de commande de la température.

Si l'on chauffe la zone située directement en amont du débouché des microcanaux, la tension de surface tendant à retenir le second fluide dans chaquemicrocanal diminue et l'effort nécessaire pour tirer une goutte de second fluide hors du microcanal est plus faible. Le chauffage directement en amont du débouché tend donc à faire diminuer la taille des gouttes.

A l'inverse, si l'on chauffe la zone située directement en aval du débouché des microcanaux, la tension de surface tendant à extraire le second fluide hors de chaque microcanal est diminuée. Le chauffage directement en aval du débouché tend donc à faire augmenter la taille des gouttes.

De façon générale, le chauffage produit les mêmes effets que l'augmentation de section au débouché des microcanaux, en ce qui concerne la formation des gouttes et leur détachement.

Selon une autre caractéristique de l'invention, les microcanaux peuvent contenir des fluides indépendants ou former des ramifications provenant d'un même canal situé en amont des microcanaux.

Selon une première variante, les microcanaux sont sensiblement parallèles les uns aux autres et débouchent d'un même côté de la chambre.

Selon une seconde variante, la chambre est de forme annulaire, les microcanaux étant agencés en étoile et débouchant en périphérie interne de la chambre.

Selon une forme de réalisation de l'invention, le dispositif comporte un corps réalisé en deux parties, les microcanaux et la chambre comportant chacun une paroi définie par l'une de ces deux parties et une autre paroi définie par l'autre de ces deux parties.

De cette manière, il est possible de faire varier les propriétés des gouttes (taille, vitesse,...) par simple changement de l'une ou de l'autre des deux parties précitées.

Ceci permet également de disposer de microcanaux de hauteur réduite et donc de former des gouttes très petites (inférieures à 10 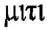 par exemple), par rapport à un corps monobloc.

L'invention sera mieux comprise et d'autres détails, caractéristiques et avantages de l'invention apparaîtront à la lecture de la description suivante faite à titre d'exemple non limitatif en référence aux dessins annexés dans lesquels :
- la figure 1 est une vue en coupe longitudinale d'un exemple de dispositif ;
- la figure 2 en est une vue en coupe transversale de ce dispositif dans laquelle les gouttes formées ne sont pas représentées ;
- la figure 3 est un diagramme représentant la taille des gouttes produites en fonction du débit du second fluide ;
- les figures 4 à 6 sont des vues schématiques en coupe transversale du dispositif, illustrant trois variantes;
- la figure 7 est une vue correspondant à la figure 2, d'un dispositif en deux parties ;
- la figure 8 est une vue en coupe longitudinale d'un exemple de dispositif;
- la figure 9 est une vue schématique d'une variante de réalisation selon l'invention dans laquelle plusieurs microcanaux agencés en parallèle débouchent dans la chambre ;
- la figure 10 est une vue schématique d'une autre variante de réalisation selon l'invention dans laquelle les microcanaux forment des ramifications débouchant dans la chambre ;

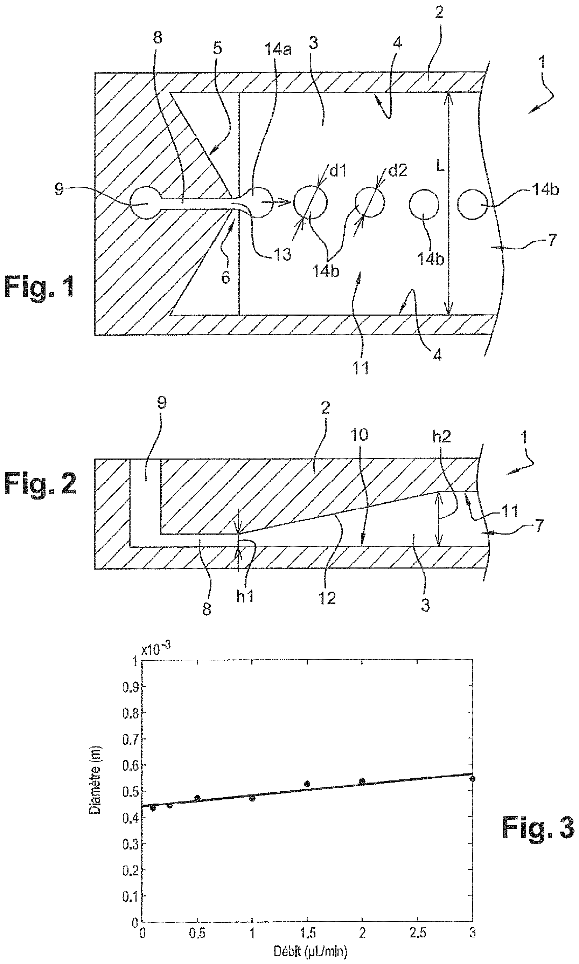

- la figure 1 1 est une vue schématique d'une autre variante de réalisation de l'invention dans laquelle la chambre est annulaire, les microcanaux étant agencés en étoile.

Les figures 1 et 2 représentent un dispositif de formation de gouttes 1 dans un circuit microfluidique, comprenant un corps 2 dans lequel est ménagée une chambre 3 délimitée par deux parois latérales 4 parallèles et opposées et par deux parois longitudinales opposées 10, 1 1. La largeur L de la chambre 3, c'est-à-dire la distance entre les deux parois latérales 4 est de l'ordre de 2 mm par exemple. La chambre 3 comporte en outre une paroi de fond 5 en forme de pointe 6 dirigée vers une extrémité opposée 7 de la chambre 3.

Le corps 2 comprend de plus un microcanal 8 dont une extrémité est reliée à un orifice de raccordement 9, notamment pour le raccordement d'une seringue ou d'une pipette, et dont l'autre extrémité débouche dans la chambre 3 au niveau de la pointe 6 de la paroi de fond 5.

La paroi longitudinale inférieure 10 de la chambre est une paroi plane et la paroi longitudinale supérieure 1 1 présente une partie oblique 12 qui s'écarte progressivement de la paroi longitudinale inférieure 10 en direction de l'extrémité opposée 7 de la chambre 3. La divergence des deux parois opposées 10, 1 1 de la chambre 3 correspond par exemple à une pente comprise entre 1 et 4 % environ d'une paroi par rapport à l'autre.

De cette manière, la section de la chambre 3 augmente progressivement de la zone dans laquelle débouche le microcanal 8 vers l'extrémité opposée 7. La hauteur minimum hl de la chambre 3, c'est-à-dire la hauteur de la chambre 3 au niveau du débouché 13 du microcanal 8, est de l'ordre de 10 à 100 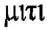 , et la hauteur maximum h2 de la chambre 3, c'est-à-dire la hauteur de la chambre 3 au niveau de son extrémité ouverte 7, est de l'ordre de 20 à 1000 µηη.

On peut associer à ce dispositif 1 des moyens pour modifier localement la tension de surface du second fluide, comprenant des moyens de chauffage du second fluide, par exemple par des électrodes intégrées au microcircuit ou en utilisant une commande externe de la température. La tension de surface diminue de façon linéaire avec la température de sorte que l'on peut pour une surface fixe changer l'énergie de surface (égale au produit de l'aire totale par la tension de surface) par chauffage par des électrodes, afin de produire les mêmes effets que l'augmentation de section au débouché du microcanal 8, avec un gradient décroissant de température à ce débouché.

Une variante de réalisation de l'invention est représentée en figure 9. Dans celle-ci, la chambre 3 présente une forme rectangulaire, et est raccordée à plusieurs microcanaux 8 sensiblement parallèles débouchant sur un même côté de la chambre 3.

Une autre variante est illustrée en figure 10, dans laquelle le dispositif comporte un réseau de microcanaux 8 comportant des ramifications, chaque ramification étant issue d'un même canal d'origine, situé en amont. Les différentes ramifications débouchent dans un même côté de la chambre 3.

Une dernière variante est visible en figure 1 1. Dans cette variante, la chambre 3 présente une forme annulaire et le dispositif comporte plusieurs microcanaux 8 agencés en étoile, s'étendant radialement depuis une même source 15 jusqu'à déboucher en périphérie interne de la chambre 3.

Ces variantes de réalisation permettent de former simultanément plusieurs trains de gouttes à l'intérieur d'une même chambre. Ceci est particulièrement utile lorsque l'on cherche à produire des populations de gouttes, contenant par exemple différents ingrédients. En fonction des besoins, les gouttes ainsi formées peuvent être manipulées ou extraites du dispositif sous la forme de mousse ou d'émulsion.

Le fonctionnement de ce dispositif de formation de gouttes va maintenant être détaillé.

La chambre 3 est remplie d'un premier fluide, par exemple de l'huile. On raccorde alors une seringue contenant un second fluide, par exemple de l'eau, à l'orifice de raccordement 9 puis on injecte de l'eau dans le microcanal 8 jusqu'à ce que celle-ci atteigne le débouché 13 du microcanal 8.

Comme indiqué précédemment, l'eau située au niveau du débouché 13 du microcanal 8 est soumise à deux forces antagonistes dues à la tension de surface. Une première force est due à un gradient d'énergie de surface qui tend à extraire l'eau hors du microcanal 8, en formant un doigt 14a qui fait saillie dans la chambre 3 et est rattaché à l'eau contenue dans le microcanal 8.

Une seconde force, opposée à la première et correspondant à la force capillaire, tend à maintenir le doigt 14a attaché à l'eau contenue dans le microcanal 8.

Le doigt 14a se détache lorsque la première force devient supérieure à la seconde force. Cette première force est fonction, pour une géométrie donnée du microcanal 8 et de la chambre 3, du volume du doigt 14a. Ainsi, en fonctionnement, le volume du doigt 14a augmente progressivement, jusqu'à ce que la première force devienne supérieure à la seconde force et que le doigt se détache pour former une goutte 14b.

Les dimensions du microcanal 8 et l'élargissement de la section de la chambre 3 sont calculés de manière à obtenir une goutte 14 d'une taille déterminée. En particulier, la hauteur hl de la chambre 3 au débouché du microcanal doit être inférieure au diamètre des gouttes 14 à former.

Les gouttes d'eau 14b sont ainsi successivement formées dans la chambre, pourvu que de l'eau soit amenée au débouché 13 du microcanal 8.

En fonction des besoins, un débit d'huile peut être imposé dans la chambre 3.

Les gouttes 14b formées au niveau du débouché 13 du microcanal 8 sont transportées naturellement en direction de l'extrémité opposée 7 de la chambre 3, en raison de l'élargissement de leur section de passage dans la chambre. En effet, comme vu ci-dessus, une goutte 14b située dans une zone de faible section, dans laquelle elle prend une forme écrasée, sera naturellement attirée par une zone de plus grande section, dans laquelle elle peut prendre une forme plus sphérique et donc moins contrainte. Comme cela est visible sur la figure 1, les gouttes 14b proches de la pointe 6 présentent un diamètre apparent dl plus grand que celui d2 des gouttes 14b proches de la seconde extrémité 7, du fait de leur écrasement entre les parois 10 et 12.

La figure 3 est un graphe représentant la variation du diamètre des gouttes 14b, mesuré à une position donnée, en fonction du débit d'eau arrivant par le microcanal 8. On remarque que cette variation est quasiment nulle même pour une grande variation du débit appliqué, ce qui montre que l'invention permet d'obtenir des gouttes 14 de taille calibrée, quelles que soit les conditions opératoires, simplifiant ainsi la mise en œuvre d'un tel dispositif de formation de gouttes. Dans l'exemple représenté en figure 3, la taille des gouttes 14b est de l'ordre de quelques centaines de micromètres mais une réduction des dimensions de ce dispositif 1 permettrait également d'obtenir des gouttes 14 d'une taille de quelques centaines de nanomètres, sans modification sensible de son fonctionnement.

Le fonctionnement du dispositif est notamment indépendant de la nature des fluides (gaz ou liquide) et de la valeur de la tension de surface.

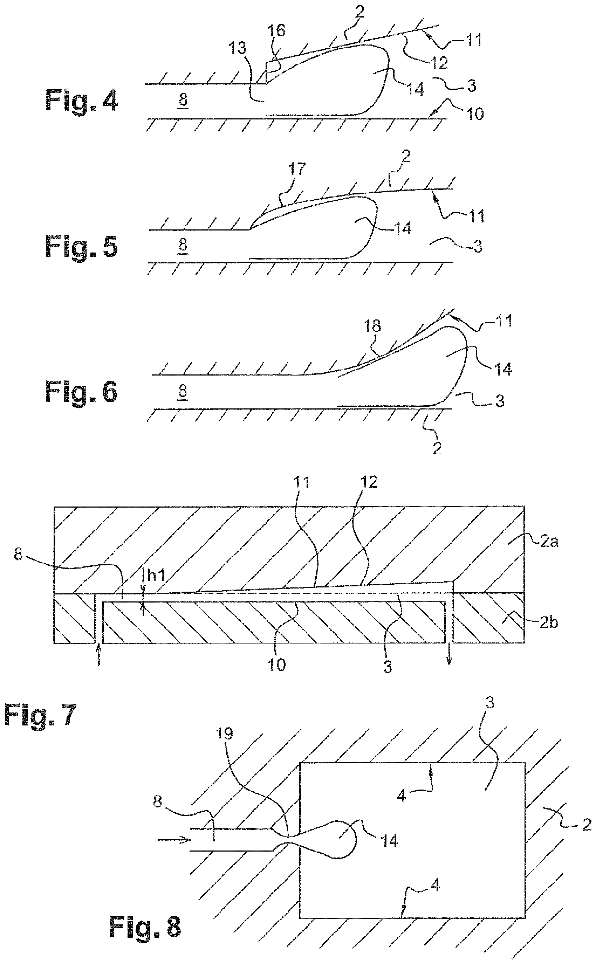

Selon une variante de réalisation représentée à la figure 4, le débouché 13 du microcanal 8 dans la chambre 3 comporte une marche 16, c'est-à-dire un élargissement brusque de la section du microcanal. Cette marche 16 est formée dans la paroi supérieure 1 1. Cette dernière comporte ainsi une partie 16 perpendiculaire au microcanal et formant la marche, prolongée par une partie oblique 12, formant, de la même manière que précédemment, un angle avec la paroi inférieure 10. Une telle marche 16 peut être utilisée pour former des gouttes plus petites, pour une pente donnée.

La figure 5 illustre une autre variante de réalisation dans laquelle la marche a été remplacée par une zone de raccordement concave 17 de la paroi supérieure 1 1, reliant le débouché 13 du microcanal 8 à la partie oblique 12.

Ceci permet de former des gouttes ou des bulles dont la vitesse de déplacement sera inférieure à celle des gouttes ou des bulles formées avec le dispositif de la figure 2.

La figure 6 illustre encore une autre variante de réalisation dans laquelle la zone de raccordement 18 de la paroi supérieure 1 1 est convexe. Cela permet de former des gouttes de taille mieux calibrée.

La figure 7 représente une forme similaire à celle de la figure 2 et dans laquelle le corps 2 est réalisé en deux parties, respectivement une partie supérieure 2a et une partie inférieure 2b. La partie oblique 12 de la paroi supérieure 1 1 de la chambre 3 est réalisée dans la partie supérieure 2a, par exemple par fraisage ou par tout autre procédé adapté. La partie inférieure 2b comporte les microcanaux 8, réalisés par exemple par photolithographie, par formage plastique ou par tout autre procédé adapté.

De cette manière, il est possible de faire varier les propriétés des gouttes (taille, vitesse, ...) par simple changement de l'une ou de l'autre des parties 2a, 2b.

Ceci permet également de disposer d'un microcanal de hauteur réduite et donc de former des gouttes très petites (inférieures à 10 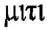 par exemple), par rapport à un corps monobloc.

Les matériaux utilisés pour les parties 2a et 2b peuvent être différents ou non. De plus, les deux parties 2a, 2b peuvent être collées ensemble, de manière indémontable, pour former un dispositif qui produit des gouttes de même taille. Elles peuvent à l'inverse être fixées l'une à l'autre de manière amovible, afin de pouvoir changer la taille des gouttes par remplacement de l'une ou l'autre des parties.

La figure 8 illustre une forme dans laquelle le microcanal comporte une zone de plus faible largeur 19 c'est-à-dire un étranglement. En variante, cette zone pourrait être un élargissement localisé (non représenté). De cette façon, la goutte 14 se détache du reste du fluide contenu dans le microcanal 8, de façon sélective et contrôlée, au niveau de l'étranglement ou de l'élargissement, ce qui permet de mieux contrôler la taille des gouttes produites.

## Revendications

1. Dispositif (1) de formation de gouttes (14) dans un circuit microfluidique, **caractérisé en ce qu'**il comprend :
- une chambre (3) contenant un premier fluide et délimitée par deux parois opposées (10, 11) qui divergent l'une par rapport à l'autre dans au moins une direction donnée, et
- une pluralité de microcanaux (8) qui contiennent chacun un second fluide et qui débouchent par un débouché (13) dans ladite chambre (3) en amont de la chambre par rapport à la direction donnée, le débouché (13) de chaque microcanal (8) dans la chambre (3) comprenant une augmentation de la section de passage du second fluide et cette augmentation provoquant la formation de gouttes (14) du second fluide et leur détachement du second fluide contenu dans le microcanal, indépendamment du débit du premier fluide et/ou du second fluide.

2. Dispositif selon la revendication 1, dans lequel les microcanaux (8) forment des ramifications provenant d'un même canal situé en amont des microcanaux (8).

3. Dispositif selon la revendication 1, dans lequel les microcanaux (8) contiennent des seconds fluides indépendants.

4. Dispositif selon l'une quelconque des revendications précédentes, dans lequel les microcanaux (8) sont sensiblement parallèles les uns aux autres et débouchent d'un même côté de la chambre (3).

5. Dispositif selon l'une quelconque des revendications 1 à 3, dans lequel la chambre (3) est de forme annulaire, les microcanaux (8) étant agencés en étoile et débouchant en périphérie interne de la chambre (3).

6. Dispositif selon la revendication 1, dans lequel dans lequel la chambre (3) est de forme annulaire, les microcanaux (8) étant agencés en étoile et radialement depuis une même source (15) jusqu'à déboucher en périphérie interne de la chambre (3).

7. Dispositif selon l'une quelconque des revendications précédentes, dans lequel la chambre (3) a une section sensiblement rectangulaire dont la hauteur est comprise entre les deux parois opposées divergentes (10, 11) et dont la longueur est supérieure à dix fois la hauteur.

8. Dispositif selon l'une quelconque des revendications précédentes, dans lequel l'une des parois (10, 11) de la chambre (3) comporte une marche (16), une partie concave (17) ou une partie convexe (18) au débouché (13) des microcanaux (8).

9. Dispositif selon l'une quelconque des revendications 1 à 8, configuré pour que le débit du premier fluide dans la chambre (3) soit réglé à une valeur déterminée.

10. Dispositif selon l'une quelconque des revendications 1 à 8, configuré pour que le débit imposé au premier fluide dans la chambre (3) soit nul.

11. Dispositif selon l'une quelconque des revendications précédentes, dans lequel la divergence des deux parois opposées (10, 11) de la chambre (3) correspond à une pente comprise entre 1 et 4 % d'une paroi par rapport à l'autre.

12. Dispositif selon l'une quelconque des revendications précédentes, comportant des moyens pour modifier localement la tension de surface des seconds fluides.

13. Dispositif selon la revendication 12, dans lequel les moyens de modification de la tension de surface des seconds fluides comportent des moyens de chauffage des seconds fluides, par exemple par un faisceau laser appliqué localement ou par des électrodes intégrées au circuit microfluidique.

14. Dispositif selon l'une quelconque des revendications précédentes, comportant un corps (2) réalisé en deux parties (2a, 2b), les microcanaux (8) et la chambre (3) comportant chacun une paroi définie par l'une des parties (2a) et une autre paroi définie par l'autre partie (2b).

## Patentansprüche

1. Vorrichtung (1) zur Bildung von Tropfen (14) in einem mikrofluidischen Schaltkreis, **dadurch gekennzeichnet, dass** sie umfasst:
- eine Kammer (3), die ein erstes Fluid enthält und durch zwei entgegengesetzte Wände (10, 11) begrenzt wird, die in mindestens eine gegebene Richtung voneinander divergieren, und
- eine Mehrzahl von Mikrokanälen (8), die jeweils ein zweites Fluid enthalten und die stromauf der Kammer in Bezug auf die gegebene Richtung durch eine Mündung (13) in die Kammer (3) münden, wobei die Mündung (13) jedes Mikrokanals (8) in die Kammer (3) eine Vergrößerung des Durchtrittsquerschnitts des zweiten Fluids umfasst und diese Vergrößerung die Bildung von Tropfen (14) des zweiten Fluids und ihr Lösen von dem im Mikrokanal enthaltenen zweiten Fluid bewirkt, unabhängig vom Volumenstrom des ersten Fluids und/oder des zweiten Fluids.

2. Vorrichtung nach Anspruch 1, bei der die Mikrokanäle (8) Verzweigungen bilden, die von demselben stromauf der Mikrokanäle (8) gelegenen Kanal ausgehen.

3. Vorrichtung nach Anspruch 1, bei der die Mikrokanäle (8) unabhängige zweite Fluide enthalten.

4. Vorrichtung nach einem der vorhergehenden Ansprüche, bei der die Mikrokanäle (8) im Wesentlichen parallel zueinander verlaufen und auf derselben Seite der Kammer (3) münden.

5. Vorrichtung nach einem der Ansprüche 1 bis 3, bei der die Kammer (3) ringförmig ist, wobei die Mikrokanäle (8) sternförmig angeordnet sind und am inneren Umfang der Kammer (3) münden.

6. Vorrichtung nach Anspruch 1, bei der bei der die Kammer (3) ringförmig ist, wobei die Mikrokanäle (8) sternförmig und radial von derselben Quelle (15) aus angeordnet sind, bis sie am inneren Umfang der Kammer (3) münden.

7. Vorrichtung nach einem der vorhergehenden Ansprüche, bei der die Kammer (3) einen im Wesentlichen rechteckigen Querschnitt hat, dessen Höhe zwischen den beiden divergierenden entgegengesetzten Wänden (10, 11) liegt und dessen Länge mehr als das Zehnfache der Höhe beträgt.

8. Vorrichtung nach einem der vorhergehenden Ansprüche, bei der eine der Wände (10, 11) der Kammer (3) eine Stufe (16), einen konkaven Teil (17) oder einen konvexen Teil (18) an der Mündung (13) der Mikrokanäle (8) aufweist.

9. Vorrichtung nach einem der Ansprüche 1 bis 8, die dazu ausgebildet ist, dass der Volumenstrom des ersten Fluids in der Kammer (3) auf einen bestimmten Wert geregelt wird.

10. Vorrichtung nach einem der Ansprüche 1 bis 8, die dazu ausgebildet ist, dass der dem ersten Fluid in der Kammer (3) aufgeprägte Volumenstrom null ist.

11. Vorrichtung nach einem der vorhergehenden Ansprüche, bei der die Divergenz der beiden entgegengesetzten Wände (10, 11) der Kammer (3) einer Neigung einer Wand in Bezug auf die andere zwischen 1 und 4 % entspricht.

12. Vorrichtung nach einem der vorhergehenden Ansprüche, die Mittel aufweist, um die Oberflächenspannung der zweiten Fluide lokal zu ändern.

13. Vorrichtung nach Anspruch 12, bei der die Mittel zum Ändern der Oberflächenspannung der zweiten Fluide Mittel zum Erhitzen der zweiten Fluide aufweisen, beispielsweise durch einen lokal aufgebrachten Laserstrahl oder durch in den mikrofluidischen Schaltkreis integrierte Elektroden.

14. Vorrichtung nach einem der vorhergehenden Ansprüche, die ein aus zwei Teilen (2a, 2b) ausgeführtes Gehäuse (2) aufweist, wobei die Mikrokanäle (8) und die Kammer (3) jeweils eine Wand aufweisen, die durch einen der Teile (2a) definiert wird, und eine weitere Wand, die durch den anderen Teil (2b) definiert wird.

## Claims

1. Device (1) for forming drops (14) in a microfluidic circuit, **characterized in that** it comprises:
- a chamber (3) containing a first fluid and delimited by two opposing walls (10, 11) which diverge from one another in at least one given direction, and
- a plurality of microchannels (8) which each contain a second fluid and which emerge through an outlet (13) in said chamber (3) upstream of the chamber with respect to the given direction, the outlet (13) of each microchannel (8) in the chamber (3) comprising an increase in the section of passage of the second fluid and this increase provoking the formation of drops (14) of the second fluid and the detachment thereof from the second fluid contained in the microchannel, independently of the flow rate of the first fluid and/or of the second fluid.

2. Device according to Claim 1, wherein the microchannels (8) form branches originating from one and the same channel situated upstream of the microchannels (8).

3. Device according to Claim 1, wherein the microchannels (8) contain second independent fluids.

4. Device according to any one of the preceding claims, wherein the microchannels (8) are substantially parallel to one another and emerge on the same side of the chamber (3).

5. Device according to any one of Claims 1 to 3, wherein the chamber (3) is of annular form, the microchannels (8) being arranged in a star configuration and emerging at the inner periphery of the chamber (3).

6. Device according to Claim 1, wherein wherein the chamber (3) is of annular form, the microchannels (8) being arranged in star configuration and radially from one and the same source (15) to emerge at the inner periphery of the chamber (3).

7. Device according to any one of the preceding claims, wherein the chamber (3) has a substantially rectangular section whose height is contained between the two divergent opposing walls (10, 11) and whose length is greater than ten times the height.

8. Device according to any one of the preceding claims, wherein one of the walls (10, 11) of the chamber (3) comprises a tread (16), a concave part (17) or a convex part (18) at the outlet (13) of the microchannels (8).

9. Device according to any one of Claims 1 to 8, configured so that the flow rate of the first fluid in the chamber (3) is set at a determined value.

10. Device according to any one of Claims 1 to 8, configured so that the flow rate imposed on the first fluid in the chamber (3) is zero.

11. Device according to any one of the preceding claims, wherein the divergence of the two opposing walls (10, 11) of the chamber (3) corresponds to a slope of between 1 and 4% of one wall with respect to the other.

12. Device according to any one of the preceding claims, comprising means for locally modifying the surface tension of the second fluids.

13. Device according to Claim 12, wherein the means for modifying the surface tension of the second fluids comprise means for heating the second fluids, for example by a laser beam applied locally or by electrodes incorporated in the microfluid circuit.

14. Device according to any one of the preceding claims, comprising a body (2) produced in two parts (2a, 2b), the microchannels (8) and the chamber (3) each comprising one wall defined by one of the parts (2a) and another wall defined by the other part (2b).
